# EUROPÄISCHE PATENTSCHRIFT

(11) **EP 0 873 297 B1**
(45) Veröffentlichungstag und Bekanntmachung des Hinweises auf die Patenterteilung: **04.07.2001**
(21) Anmeldenummer: 96938171.4
(22) Anmeldetag: 11.11.1996
(51) Int. Cl.: C07C 67/03, C07C 69/06, C07C 69/10, C07C 69/675, C08F 8/12, C08F 18/06, C08G 65/20, C07C 31/22, C07C 27/02

(54) **VERFAHREN ZUR HERSTELLUNG VON HYDROXYHALTIGEN VERBINDUNGEN AUS AMEISENSÄUREESTERN**
PROCESS FOR PREPARING HYDROXY-CONTAINING COMPOUNDS FROM FORMIC ACID ESTERS
PROCEDE DE PREPARATION DE COMPOSES CONTENANT DE L'HYDROXY, ISSUS D'ESTERS D'ACIDE FORMIQUE

(30) Priorität: 10.11.1995 DE 19542035
(43) Veröffentlichungstag der Anmeldung: 28.10.1998
(73) Patentinhaber: BASF AKTIENGESELLSCHAFT, 67056 Ludwigshafen (DE)
(72) Erfinder: KRATZ, Detlef, D-69121 Heidelberg (DE); SIGWART, Christoph, D-69198 Schriesheim (DE)
(74) Vertreter: Isenbruck, Günter, Dr.
(86) Internationale Anmeldenummer: EP9604923
(87) Internationale Veröffentlichungsnummer: WO9717319

(56) Entgegenhaltungen:
- EP-A- 0 289 921
- DE-A- 4 236 971

## Beschreibung

Die Erfindung betrifft ein Verfahren zur Herstellung von hydroxyhaltigen Verbindungen (1), insbesondere Alkoholen, und Ameisensäureestern einer hydroxyhaltigen Verbindung (2) durch Umesterung eines Ameisensäureesters (im folgenden auch "Formiat") einer hydroxyhaltigen Verbindung (1) mit einer hydroxyhaltigen Verbindung (2), insbesondere Alkohol, in Gegenwart bestimmter tertiärer Amine.

Es ist bekannt, Ester R¹COOR³ und/oder hydroxyhaltige Verbindung, insbesondere Alkohol R²OH, durch Umesterung von Estern R¹COOR² mit hydroxyhaltiger Verbindung, insbesondere Alkohol R³OH, gemäß folgendem Reaktionsschema herzustellen:

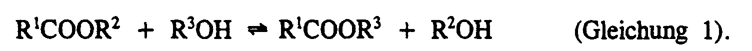

Derartige Umesterungsreaktionen sind beispielsweise in "The Chemistry of Carboxylic Acids and Esters, Ed. S. Patai, 1969, S. 103 ff; Methoden der organischen Chemie (Houben-Weyl), Band E5, Georg-Thieme Verlag, Stuttgart 1985, Seite 702 ff" beschrieben.

Für den Fall, daß R¹ = H ist, liegen Ester der Ameisensäure vor.

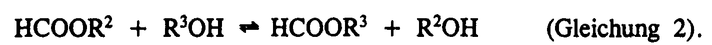

Einige ausgewählte Reaktionen, bei denen Ameisensäureester entstehen bzw. in technischen Prozessen anfallen und anschließend nach Gleichung 2 umgeestert werden, sind im nachfolgenden aufgeführt.
1. Ameisensäureester werden in der synthetischen Chemie als Schutzgruppe für Alkohole eingesetzt. Zur Gewinnung des geschützten Alkohols muß anschließend die Schutzgruppe quantitativ entfernt werden.
2. Ameisensäureester treten in großtechnischen Prozessen auf, bei denen Ameisensäure als saurer Katalysator verwendet wird, und bei denen Alkohole entstehen oder zugegen sind. Besonders bei hochsiedenden Verbindungen oder Polymeren sind diese Ester oftmals schwer durch einen physikalischen Reinigungsprozeß wie Umkristallisation, Extraktion oder Destillation von den gewünschten Alkoholen abzutrennen, da ihre physikalischen Eigenschaften denen der freien Alkohole sehr ähnlich sind. Daher wird zur Reinigung beispielsweise die chemische Methode der Umesterung angewendet. Die Umesterung muß quantitativ verlaufen, und Katalysatorrückstände dürfen nicht im Produkt verbleiben.
   a) Beispielsweise wird bei der kationischen Polymerisation von Tetrahydrofuran (THF) in Gegenwart von Ameisensäure Polyoxybutylenglykolformiat (PolyTHF-Formiat) gebildet (EP-A-503 386). Um reines Polyoxybutylenglykol (PolyTHF) zu erhalten, muß die Formiat-Gruppe quantitativ entfernt werden.
   b) Eine weitere Anwendung in der Polymerchemie ist die Polymerisation von Vinylformiat zu Poly(vinylformiat). Um das großtechnische Produkt Poly(vinylalkohol) zu erhalten, müssen die in äquimolaren Mengen vorhandenen Formiatgruppen quantitativ entfernt werden.
   c) Bei der durch Ameisensäure katalysierten Prins-Reaktion von Olefinen mit Formaldehyd entstehen in großer Menge die Formiate der gebildeten Alkohole (D.R. Adams, S.P. Bhatnagar, Synthesis (1977) 661-672; J.S. Bajorek, R. Battaglia, G. Pratt, J.K. Sutherland, J. Chem. Soc. Perkin I (1974) 1243-1245). Um die Alkohole zu erhalten, muß der Ester im Laufe der Aufarbeitung gespalten werden.
3. Bei Reaktionen, die in Gegenwart von Formaldehyd durchgeführt werden, bildet sich häufig durch Cannizzaro-Reaktion von zwei Äquivalenten Formaldehyd ein Äquivalent Methanol und ein Äquivalent Ameisensäure. Ameisensäure entsteht auch durch gekreuzte Cannizzaro-Reaktion von Aldehyden mit Formaldehyd. So entstandene Ameisensäure bildet leicht unter den Reaktionsbedingungen als Nebenprodukt unerwünschte Ameisensäureester. Auch hier gilt es, die Formiate entweder durch physikalische Trennmethoden bzw. durch chemische Reaktion zu entfernen.

Die Reaktionen nach Gleichung 1 und Gleichung 2 sind Gleichgewichtsreaktionen. Ausgehend von dem Ester R¹COOR² kann das Gleichgewicht zugunsten des gewünschten Produktes, dem Ester R¹COOR³ bzw. dem Alkohol R²OH, verschoben werden, indem entweder eine Ausgangskomponente im Überschuß verwendet wird oder bevorzugter eine Reaktionskomponente, der entstehende Alkohol R²OH oder Ester R¹COOR³, z.B. destillativ oder durch Kristallisation aus dem Gleichgewicht entfernt wird.

Es ist bekannt, daß zur Durchführung der Umesterung nach Gleichung 1 oder Gleichung 2 der Zusatz eines Katalysators erforderlich ist. Als typische Umesterungskatalysatoren werden in der Technik u.a. Schwefelsäure, p-Toluolsulfonsäure, Natronlauge, Natriumalkoholate, Aluminiumalkoholate, Kaliumcyanid wie auch saure oder basische Ionenaustauscher verwendet. Diese Katalysatoren weisen jedoch eine Reihe von Nachteilen auf: (siehe: The Chemistry of Carboxylic Acids and Esters, Ed. S. Patai, 1969, S. 103 ff; Methoden der organischen Chemie (Houben-Weyl), Band E5, Georg-Thieme Verlag, Stuttgart 1985, Seite 702 ff).
1. Die als Katalysator verwendeten starken Basen und Säuren können zu zahlreichen unerwünschten Nebenreaktionen wie Eliminierung, C-Alkylierung oder Polymerisation führen.
2. Falls das gewünschte Produkt der Alkohol R²OH ist, kommt es bei Verwendung von stark basischen anorganischen Verbindungen wie Natriummethanolat als Katalysator zu Ausbeuteverlusten, da ein Teil des Alkohols als Alkoholat gebunden bleibt.
3. Für die Abtrennung des Katalysators bzw. Freisetzung von Alkoholen aus ihren Alkoholaten muß neutralisiert werden. Dadurch entstehen anorganische Salze, die vom gewünschten Produkt abgetrennt werden müssen. Der Katalysator wird dadurch zerstört. Das anorganische Salz muß entsorgt werden.
4. Die Isolierung des Wertproduktes bereitet insbesondere dann Schwierigkeiten, wenn der gewünschte Alkohol oder Ester hochsiedend, kristallin oder ein Polymer ist. Das entstandene anorganische Salz kann in diesem Fall nicht immer problemlos abgetrennt werden. Destillation in Gegenwart eines anorganischen Salzes führt zur Zersetzung des gewünschten Alkohols oder Esters.
5. Werden saure oder basische Ionenaustauscher als Katalysator eingesetzt, so ist eine Reaktionsführung bei höheren Temperaturen nicht möglich, da Ionenaustauscher in der Regel bei Temperaturen größer 60-100°C zersetzt werden. Andererseits kann auch hier eine Reaktionskomponente, z.B. der Alkohol R²OH, am festen Träger gebunden bleiben, wodurch Ausbeuteverluste bedingt sind. Die Standzeit von Ionenaustauschern ist begrenzt. Ein weiterer Nachteil heterogener Katalysatoren ist, daß diese für die Reaktion mit schwerlöslichen Verbindungen, insbesondere Polymeren, ungeeignet sind.
6. Wird die Umesterungsreaktion in Gegenwart von Wasser durchgeführt, so wird als Konkurrenzreaktion die Verseifung des Esters unter Bildung von Alkohol und anorganischem Salz der Säure - im Falle der Ameisensäure z.B. von Natriumformiat - beobachtet. Die destillative Abtrennung von Alkoholen in Gegenwart von Ameisensäureestern führt bekanntermaßen zu Ausbeuteverlusten durch Zersetzung. Wasser bewirkt außerdem bei Verwendung anorganischer Alkoholate als Katalysator die Bildung der freien Base und damit Desaktivierung des eigentlichen Katalysators. Beispielsweise bildet sich aus Natriumalkoholat und Wasser die als Katalysator weitaus weniger wirksame Natronlauge.

In der EP-A-0 289 921 ist beispielsweise die Umesterungsreaktion von Trimethylolalkanformiat mit Methanol beschrieben. Als Katalysatoren können Alkali- oder Erdalkalialkoholate verwendet werden. Zur Gewinnung des gewünschten Alkohols TMP muß vor der weiteren Aufarbeitung der Katalysator entweder durch Ionenaustauscher oder durch Neutralisation mit einer Säure entfernt werden. Dadurch wird der Katalysator zerstört und der Aufarbeitungsprozeß verteuert sich. Als Nebenprodukt fällt ein anorganisches Salz, z.B. Natriumacetat oder -chlorid, an, das entsorgt werden muß.

Auch die Verwendung tertiärer Amine als Umesterungskatalysatoren ist für spezielle Reaktionen bekannt.

In der DE-OS 24 60 039 ist die Umesterung von Acetoxymethylpyridinen mit Methanol zu Hydroxymethylpyridinen und Methylacetat in Gegenwart des tertiären Amins Triethylamin beschrieben. Der gebildete Alkohol Hydroxymethylpyridin ist durch elektronenziehende Gruppen stabilisiert. Die in der DE-OS 24 60 039 offenbarte Umesterungsreaktion ist dadurch eingeschränkt, daß nur ein ausgewählter aktivierter Essigsäureester mit primärer Alkoholfunktionalität und Pyridinsubstituenten mit Methanol umgesetzt werden kann.

Die Reaktionsträgheit von nicht aktivierten Acetaten bei der Umesterung mit Methanol unter Verwendung tertiärer Aminbasen als Katalysator ist experimentell bestätigt worden. Bei dem Versuch, ein Gemisch der Mono-, Di- und Triacetate des 1,1,1-Trimethylolpropans (TMP) unter den in der DE-OS 24 60 039 beschriebenen Bedingungen zu TMP und Methylacetat umzuestern, konnte kein Umsatz zu Methylacetat festgestellt werden. Ebenso gelingt die Reaktion nicht durch Zugabe einer stärkeren Base mit Amidinstruktur wie beispielsweise 1,8-Diazabicyclo[5.4.0]undec-7-en (DBU). Erst nach Zugabe von methanolischem Tetramethylammoniumhydroxid, also von OH⁻Ionen, beobachtet man die Bildung von Methylacetat, allerdings mit sehr unbefriedigendem Umsatz.

Ein weiterer Nachteil des in der DE-OS 24 60 039 beschriebenen Verfahrens ist, daß die Umesterung mit Ethanol praktisch gar keine Reaktion liefert und mit Isopropanol gänzlich versagt.

In der EP-A-0 168 167 ist die Umesterung von Ethylacetat und Propylencarbonat mit Methanol unter Verwendung eines heterogenen aminhaltigen Katalysators offenbart. Das Katalysatorsystem besteht aus einer organischen Verbindung, welche ein VA-Gruppenelement enthält, und einem unlöslichen Feststoff als Träger. Die das VA Gruppenelement enthaltende organische Verbindung kann ein Amin oder Phosphin sein. Beispielsweise ist die organische Verbindung ein Amidin, Guanidin, Mono-, Di- oder Trialkylamin.

Die Ausbeute an Methylacetat aus der Umesterung von Ethylacetat mit Methanol bei 24 Stunden Reaktionszeit beträgt lediglich 27%. Über die Standzeit des heterogenen Trägers sind keine Angaben gemacht.

Weitere Nachteile des in der EP-A-0 168 167 offenbarten Verfahrens sind die komplizierte Herstellung des Katalysators und die Beschränkung der Anwendung wegen der heterogenen Reaktionsführung auf spezielle im Reaktionsmedium lösliche Substrate.

DE-A-4 236 971 betrifft ein Verfahren zur Formylierung von Alkoholen und Aminen. Dabei werden beispielsweise primäre oder sekundäre aliphatische Alkohole mit Ameisensäurecyanmethylester umgesetzt, wobei durch Umesterung Ameisensäureester der primären oder sekundären Alkohole erhalten werden. Die Umsetzung kann beispielsweise in Gegenwart von hererocyclischen Aminen wie Imidazol oder 4-Dimethyl-aminopyridin durchgeführt werden. Bei beiden Verbindungen handelt es sich um heterocyclische Stickstoffverbidnungen, die mindestens zwei Doppelbindungen im heterocyclischen Kern enthalten.

Aufgabe der vorliegenden Erfindung ist es, ein Verfahren zur Herstellung von hydroxyhaltigen Verbindungen, insbesondere Alkoholen, und Ameisensäureestern durch Umesterung von Ameisensäureestern bereitzustellen, das die beschriebenen Nachteile und Einschränkungen vermeidet.

Diese Aufgabe wird gelöst durch ein Verfahren zur Herstellung von hydroxyhaltigen Verbindungen und Ameisensäureestern durch Umesterung, bei dem man Ameisensäureester einer organischen hydroxyhaltigen Verbindung 1 und eine organische hydroxyhaltige Verbindung 2 in Gegenwart eines tertiären Amins, ausgewählt aus tertiären Aminen
a) der allgemeinen Strukturformel R^{a}R^{b}R^{c}N, wobei die Reste R^{a}, R^{b}, R^{c} gleich oder unterschiedlich sein können und jeweils unabhängig voneinander einen verzweigten oder nicht verzweigten Alkylrest mit vorzugsweise 1 bis 12, bevorzugter 1 bis 6, insbesondere 1 bis 3 Kohlenstoffatomen, Cycloalkylrest mit vorzugsweise 3 bis 12, insbesondere 6 bis 12 Kohlenstoffatomen oder aromatischen Kohlenwasserstoffrest mit vorzugsweise 6 bis 12 Kohlenstoffatomen bedeuten, welche gewünschtenfalls substituiert sein können, oder
b) heterocyclischen tertiären Stickstoffverbindungen, ausgewählt aus Heterocycloalkan mit vorzugsweise 3 bis 12 Kohlenstoffatomen und 1 bis 3 Stickstoffatomen
als Umesterungskatalysator unter Bildung der organischen hydroxylhaltigen Verbindung 1 und Ameisensäureester der hydroxylhaltigen Verbindung 2 umestert, wobei Verbindungen 1 und 2 voneinander verschieden sind.

Beispielsweise werden hydroxyhaltige Verbindungen R²OH und Ameisensäureester HCOOR³ dadurch hergestellt, daß man Ameisensäureester der Formel HCOOR² und eine Verbindung der Formel R³OH in Gegenwart eines tertiären Amins als Umesterungskatalysator unter Bildung von Ameisensäureester der Formel HCOOR³ und einer Verbindung der Formel R²OH umestert, wobei R² und R³ voneinander verschiedene organische Reste sind. Die Reste R² und R³ sind unter den Reaktionsbedingungen inerte organische

Substituenten; sie sind für die Durchführbarkeit des Verfahrens nicht kritisch.

Bevorzugte Ausführungsformen sind nachstehend beschrieben.

Die Vorteile des Verfahrens gemäß der Erfindung sind vielfach.
1. Die Reaktion verläuft mit hoher Selektivität und hoher Ausbeute bezüglich der Ameisensäureester.
2. Die Reaktionsbedingungen und Reaktionstemperaturen sind milde. Es werden sehr milde Basen verwendet und damit Nebenreaktionen vermieden.
3. Ein Verlust an Katalysator tritt im Unterschied zu den zuvor beschriebenen, aus dem Stand der Technik bekannten anorganischen und heterogenen Katalysatoren nicht auf. Die Wiedergewinnung und Rückführung ist einfach.
4. Die Aufarbeitung der Reaktionsgemische erfolgt in einfacher Weise durch Destillation oder Kristallisation. Ein Neutralisationsschritt ist nicht notwendig.
5. Die Reaktion gelingt auch in Gegenwart von Wasser quantitativ.

Die für die Umesterung verwendete hydroxyhaltige Verbindung (2) wird in bezug auf die Hydroxylgruppen vorzugsweise in mindestens stöchiometrischer Menge in bezug auf die Ameisensäureestergruppen zum Ameisensäureester der hydroxylhaltigen Verbindung (1) gegeben.

Die Umesterung des Ameisensäureesters aus einer organischen hydroxyhaltigen Verbindung (1), z.B. R²OH, insbesondere Alkohol, und Ameisensäure (bezüglich der Hydroxylgruppen) mit mindestens äquimolaren Mengen einer anderen organischen hydroxyhaltigen Verbindung (2) (bezüglich der Estergruppierung), insbesondere Alkohol R³OH, vorzugsweise in einem Molverhältnis Hydroxygruppe zu Estergruppe von 1:10, insbesondere 1:5, in Gegenwart eines tertiären Amins gelingt schnell und mit quantitativer Ausbeute und ist nicht wie die aus EP-A-0 289 921 bekannte Reaktion auf bestimmte Ameisensäureester beschränkt. Beispielsweise können gemäß einer Ausführungsform der Erfindung die Formiate des Trimethylolpropans mit Methanol in Gegenwart von Triethylamin quantitativ zu TMP und Methylformiat umgesetzt werden. Gleichermaßen reagieren die Ameisensäureester von sekundären, tertiären und aromatischen Alkoholen wie Cyclohexylformiat, tert.-Butylformiat, Phenylformiat nach Zugabe von Methanol und tertiärer Aminbase zum Alkohol und Methylformiat.

Die organische hydroxylhaltige Verbindung (1) kann eine oder mehrere Hydroxylgruppen aufweisen, vorzugsweise 1 bis 6, besonders bevorzugt 1 bis 3 Hydroxylgruppen. Es kann sich auch um ein hydroxylgruppenhaltiges Polymer handeln, z.B. um einen Polyvinylalkohol. Sie kann die Formel R²OH aufweisen.

Der R²-Rest des umzuesternden Ameisensäureesters der Formel HCOOR² ist dabei im allgemeinen ein gesättigter oder ungesättigter, verzweigter oder nicht verzweigter, linearer, ggf. substituierter Kohlenwasserstoffrest mit vorzugsweise 1 bis 30 Kohlenstoffatomen, insbesondere 2 bis 20 Kohlenstoffatomen, besonders bevorzugt 3 bis 18 Kohlenstoffatomen, welcher gewünschtenfalls Ethergruppe(n), Estergruppe(n), Hydroxygruppe(n) oder Ketongruppe(n) enthält, oder
ein aromatischer Kohlenwasserstoffrest mit vorzugsweise 6 bis 12 Kohlenstoffatomen, welcher gewünschtenfalls substituiert ist, oder
ein Cycloalkylrest mit vorzugsweise 3 bis 12, insbesondere 6 bis 12 Kohlenstoffatomen ist, welcher gewünschtenfalls substituiert ist, oder
ein heterocyclischer Rest mit vorzugsweise 3 bis 12 Kohlenstoffatomen und 1 bis 3 Stickstoffatomen, welcher gewünschtenfalls substituiert ist, wobei die Substituenten aus Benzyl, Phenyl, Cyclohexyl, Pyridyl, Methylpyridyl, Hydroxy, Carboxyl, Alkyl, letztere mit vorzugsweise 1 bis 12 Kohlenstoffatomen, ausgewählt sind.

Besonders bevorzugt ist für R² Propyl, tert.-Butyl, Trimethylolpropanrest, Cyclohexyl, Phenyl, Hydroxypivalinsäureneopentylglykolesterrest, Polyoxybutylenrest, Di-trimethylolpropanrest oder Methylpyridyl.

Der Rest R² des umzuesternden Ameisensäureesters der Formel HCOOR² kann auch durch weitere Reste funktionalisiert sein.

Die als Umesterungskatalysatoren verwendeten tertiären Amine sind z.B. Verbindungen der allgemeinen Strukturformel R^{a}R^{b}R^{c}N. Die Reste R^{a}, R^{b}, R^{c} sind gleich oder unterschiedlich. Vorzugsweise bedeuten sie jeweils unabhängig voneinander einen verzweigten oder nicht verzweigten Alkylrest mit vorzugsweise 1 bis 12, bevorzugt 1 bis 6, am bevorzugtesten 1 bis 3 Kohlenstoffatomen, Cycloalkylrest mit vorzugsweise 3 bis 12, insbesondere 6 bis 12 Kohlenstoffatomen oder aromatischen Kohlenwasserstoffrest mit vorzugsweise 6 bis 12 Kohlenstoffatomen, welche gewünschtenfalls substituiert sein können.

Das tertiäre Amin kann auch eine heterocyclische tertiäre Stickstoffverbindung ausgewählt aus Heterocycloalkan. mit vorzugsweise 3 bis 12 Kohlenstoffatomen und 1 bis 3 Stickstoffatomen sein.

Vorzugsweise wird als heterocyclische Stickstoffverbindung 1,4-Diazabicyclooctan, N,N'-Dialkylpiperazin, N-Alkylpiperidin oder N-Alkylaziridin verwendet.

Der Alkylrest in den zuvor genannten Verbindungen ist vorzugsweise ein verzweigter oder nicht verzweigter, linearer Alkylrest mit vorzugsweise 1 bis 6, am bevorzugtesten 1 bis 4 Kohlenstoffatomen.

Der pKₐ-Wert des tertiären Amins sollte größer als 5 sein, bevorzugt zwischen 7 und 12 liegen. Die Wahl des Amins kann problemlos auf die anderen Reaktionskomponenten abgestimmt werden. Bevorzugt werden solche Amine verwendet, die einen Siedepunkt haben, der oberhalb des Siedepunktes des gebildeten Ameisensäureesters und des Siedepunktes der eingesetzten hydroxyhaltigen Verbindung (2), insbesondere Alkohol, liegt. Wird mit Methanol umgeestert, so eignen sich Amine mit einem Siedepunkt, der über dem des Methylformiats und dem des Methanols liegt, insbesondere Trialkylamine wie Triethylamin und Tributylamin.

Vorzugsweise ist der Umesterungskatalysator trägerfrei bzw. homogen. Der Umesterungskatalysator wird bevorzugt in flüssiger Form zugesetzt.

Das tertiäre Amin kann in katalytischen Mengen von 0,01 bis 10 Mol%, bevorzugt von 0,1 bis 5 Mol%, bezogen auf Formiatgruppe als 100 Mol%, hinzugegeben werden. Ein Überschuß an tertiärem Amin, der über die vorhandene Menge an Ameisensäureäquivalenten hinausgeht, bringt keine Nachteile, da nach beendeter Umesterung alle Reaktionskomponenten wiedergewonnen werden. Beispielsweise lassen sich leichtflüchtige Amine wie Trimethyl- oder Triethylamin einfach durch Destillation von dem hochsiedenden Alkohol abtrennen und wiederverwerten. Im Falle, daß das verwendete Amin höher als der Alkohol (1) bzw. R²OH siedet, kann umgekehrt verfahren werden. Zuerst wird der gewünschte Alkohol (1) bzw. R²OH durch Destillation vom tertiären Amin abgetrennt. Das verbleibende tertiäre Amin muß nicht aufgearbeitet werden, da der aminhaltige Rückstand direkt wieder zur Reaktion eingesetzt werden kann. In bestimmten Fällen ist ein Überschuß an Aminkomponente von Vorteil, insbesondere dann, wenn sich das Amin als Lösungsmittel für die Reaktion eignet.

Als organische hydroxyhaltige Verbindungen (2) werden bei der Umesterung beispielsweise ein-, zwei-, dreiwertige oder höherwertige Alkohole mit bis zu 6 OH-Gruppen verwendet, z.B. der allgemeinen Formel R³OH.

Gemäß einer bevorzugten Ausführungsform ist der Rest R³ des für die Umesterung verwendeten Alkohols R³OH ein gesättigter oder ungesättigter, verzweigter oder nicht verzweigter linearer Kohlenwasserstoffrest mit vorzugsweise 1 bis 20 Kohlenstoffatomen, insbesondere 1 bis 8 Kohlenstoffatomen, besonders bevorzugt 1 bis 3 Kohlenstoffatomen, ein aromatischer Kohlenwasserstoffrest mit vorzugsweise 6 bis 12 Kohlenstoffatomen oder ein Cycloalkylrest mit vorzugsweise 3 bis 12, insbesondere 6 bis 12 Kohlenstoffatomen.

Beispiele des für die Umesterung verwendeten Alkohols R³OH sind Cyclohexanol, Methanol, Ethanol, Propanol, Butanol, Isopropanol, Isobutanol, tert.-Butanol und Benzylalkohol.

Die Alkohole R²OH und R³OH können primär, sekundär oder tertiär sein. Die Reste R² und R³ können weitere OH-Gruppen tragen, und damit kann der Ester HCOOR² oder HCOOR³ auch mehr als eine Ameisensäureestergruppe aufweisen.

In der nachstehenden Ausführung wird Verbindung (1) am Beispiel R²OH und Verbindung (2) am Beispiel R¹OH erläutert. Die Ausführungen gelten entsprechend für (1) und (2).

Die Anwendungsbreite der Umesterungsreaktion ist von den physikalischen Eigenschaften der Reaktionskomponenten abhängig. Es kann vorteilhaft sein, wenn der Alkohol R²OH und der Ester HCOOR² höher sieden als der zur Umesterung verwendete Alkohol R³OH und der daraus entstehende Ester HCOOR³, also eine Abstufung der Siedepunkte HCOOR² ≈ R²OH > R³OH ≥ HCOOR³ vorliegt.

Als bei der Umesterung einzusetzende Alkohole R³OH eignen sich für die Herstellung reinerer Produkte solche, die leichter flüchtig sind als die gebildete hydroxylhaltige Verbindung, beispielsweise der Alkohol R²OH. R³ kann eine primäre, sekundäre oder tertiäre Funktion darstellen. Falls R³OH weniger flüchtig ist als R²OH, so entstehen Produktgemische.

Besonders geeignet sind solche Alkohole, deren Siedepunkt höher als der des entsprechenden gebildeten Ameisensäureesters HCOOR³ ist, so daß der im Laufe der Umesterung gebildete Ameisensäureester durch Destillation aus dem Gleichgewicht entfernt werden kann. Dadurch kann ein hoher Überschuß an Alkohol vermieden werden. Ebenso sollte der Alkohol R³OH möglichst kein Azeotrop mit dem Ameisensäureester bilden. Die Umesterung gelingt jedoch selbst in einem derartigen Fall durch gemeinsames Entfernen von leichtsiedendem Alkohol und Ameisensäureester. Alkohole wie Methanol, Ethanol, Propanol, Butanol, Isopropanol, Isobutanol, tert.-Butanol eignen sich besonders. Methanol wird bevorzugt eingesetzt, da hier eine große Siedepunktsdifferenz zwischen Alkohol und Ameisensäureester besteht. Zur quantitativen Durchführung der Reaktion wird R³OH in mindestens stöchiometrischen Mengen bezüglich der Ameisensäureäquivalente eingesetzt. In bestimmten Fällen ist es vorteilhaft, die Menge des Alkohols R³OH zu erhöhen, da diese dann zugleich als Lösungsmittel für den umzuesternden Ester HCOOR² oder den gebildeten Alkohol R²OH dient. Falls bei der Umesterung ein kristalliner und in dem Reaktionsmedium schwer löslicher Alkohol R²OH entsteht, kann letzterer nach der Reaktion direkt aus R³OH oder dem Amin durch Kristallisation isoliert werden. Im Falle eines polymeren Alkohols kann dieser aus R³OH beispielsweise ausgefällt werden.

Gemäß einer bevorzugten Ausführungsform wird bei der Erfindung ein Verfahren zur Herstellung von Alkoholen durch Umesterung eines Ameisensäureesters nach Gleichung 2, wobei der Alkohol R²OH und der Ester HCOOR² höher sieden als der zur Umesterung verwendete Alkohol R³OH und der daraus entstehende Ester HCOOR³, zur Verfügung gestellt.

Die Umesterungsreaktion kann entweder kontinuierlich oder diskontinuierlich durchgeführt werden. Die verwendete Reaktionstemperatur ist von den Siedepunkten der Reaktionskomponenten abhängig. Die Umesterung kann unter vermindertem Druck, bei Atmosphärendruck oder Überdruck durchgeführt werden. Gemäß einer bevorzugten Ausführungsform mit Methanol als Umesterungsreagenz und Triethylamin als Katalysator wird der Ameisensäureester HCOOR² bei Atmosphärendruck bei einer Temperatur von 35 bis 65°C umgesetzt. Dabei beträgt das molare Verhältnis der eingesetzten Reaktionskomponenten HCOOR²/Methanol/Triethylamin 1/1-10/0.0005-0.1, insbesondere etwa 1/5/0,05. Die Menge an Methanol kann jedoch bis auf die dem Formiat entsprechende Menge reduziert werden. Das gebildete Methylformiat wird kontinuierlich in einer gebräuchlichen Destillationsapparatur abdestilliert. Zur kontinuierlichen Durchführung eignet sich eine Gegenstromkolonne.

Die Umesterung kann auch in Gegenwart weiterer inerter Lösungsmittel durchgeführt werden, die zur Homogenisierung der Reaktionskomponenten zugesetzt werden. Beispielsweise können acyclische und cyclische Ether wie Tetrahydrofuran, Dioxan und Diethylether oder aromatische Verbindungen wie Toluol als auch gegenüber dem Reaktionsmedium stabile Ester wie Ethylacetat eingesetzt werden.

Ein weiterer Vorteil des Verfahrens gemäß der Erfindung ist, daß die Umesterung von Ameisensäureestern HCOOR² auch vollständig in Gegenwart von Wasser durchgeführt werden kann.

Im Unterschied hierzu verläuft die Umesterung von Ameisensäureestern des Trimethylolpropans (TMP) mit einem 1:1-Gemisch aus Methanol und Wasser bezogen auf das Volumen mit 10 Mol%-iger Natronlauge als Katalysator nicht quantitativ. Durch Verseifung wird Natriumformiat gebildet, und auch bei längerem Behandeln mit Methanol wird nur ein Umsatz von maximal 75% bezüglich der TMP-Formiate beobachtet. Bei der destillativen Isolierung des TMP stört zudem das gebildete Natriumformiat.

Die Anwendungsbreite der gefundenen Umesterungsreaktion soll an Beispielen veranschaulicht werden.

Die erfindungsgemäße Umesterungsreaktion der Ameisensäureester verläuft selektiv bezüglich der Formiatfunktionalität in Gegenwart anderer Esterfunktionen. Mono- und Diameisensäureester von Hydroxypivalinsäureneopentylglykolester (HPN), die als Nebenprodukte bei der großtechnischen Produktion von HPN, wie sie z.B. in DE 19 58 463 oder DE 22 33 357 beschrieben ist, entstehen, können selektiv umgesetzt werden. Erfindungsgemäß gelingt die selektive Umesterung dieser Mono- und Diameisensäureester von HPN mit einem Gemisch aus Methanol und Triethylamin zu HPN und Methylformiat ohne Spaltung der zentralen Estergruppe des HPN. Nach Entfernung von überschüssigem Methanol/ Triethylamin wird HPN ohne Verluste erhalten. Bei Verwendung der typischen "anorganischen" Umesterungskatalysatoren wie beispielsweise Natriummethanolat wird hingegen die Bildung von Hydroxypivalinsäuremethylester und Neopentylglykol beobachtet.

Auch Ameisensäureester polymerer Verbindungen lassen sich sehr gut umestern. Beispielsweise kann Polytetrahydrofuran (Poly-THF), das an den alkoholischen Endgruppen durch Ameisensäure verestert ist (ein Produkt, das bei der THF-Polymerisation mit W-Katalysatoren und Ameisensäure (EP-A-0 503 394) entsteht), mit Methanol und Triethylamin als Katalysator nach einer Ausführungsform quantitativ zu Poly-THF umgesetzt werden. Die Isolierung des gereinigten Poly-THF erfolgt in einfacher Weise durch Abdestillieren von Methanol und Triethylamin.

Überraschenderweise werden bei Ersatz der anorganischen Base Natronlauge durch ein tertiäres Amin wie beispielsweise Triethylamin die Formiate des TMP zu TMP annähernd quantitativ umgesetzt. Auch hier wird teilweise die Verseifung zu Ameisensäure, die als Triethylammoniumformiat gelöst vorliegt, beobachtet. Die Reaktion verläuft jedoch auch bei einem pH-Wert < 7 quantitativ. Damit ist die Katalyse durch ein tertiäres Amin wie Triethylamin der Katalyse durch Natronlauge überlegen. Zudem bietet die durch ein tertiäres Amin katalysierte Reaktion Vorteile durch vereinfachte Aufarbeitung, da sich Alkohole auch in Gegenwart von organischen Ameisensäuresalzen unzersetzt destillieren lassen.

Die Erfindung wird anhand der nachfolgenden Beispiele näher erläutert.

### Vergleichsbeispiel 1

Dieses Vergleichsbeispiel zeigt die Reaktionsträgheit von nicht aktivierten Acetaten bei der Umesterung mit Methanol.

132 g (0,75 Mol) eines Gemisches aus Trimethylolpropan (TMP), TMP-monoacetat, TMP-diacetat und TMP-triacetat (GC-Flächen % = 32/50,5/16/1,5) werden in 240 g Methanol (7,5 Mol) gelöst. Nach Zugabe von 38,5 g (0,38 Mol) Triethylamin wird zum Rückfluß erhitzt. Am Kolonnenkopf wird eine Temperatur von 65°C beobachtet, Methylacetat kann nicht nachgewiesen werden. Das prozentuale Verhältnis der Acetate im Sumpf bleibt unverändert.

Das gesamte Methanol und Triethylamin werden abdestilliert, und der Reaktionsmischung werden 200 ml Methanol und 22 g 1,8-Diazabicyclo[5.4.0]undec-7-en (DBU) zugesetzt. Nach Erwärmen zum Rückfluß wird kein Umsatz der Acetate beobachtet. Die Kolonnenkopftemperatur beträgt 65°C.

Schließlich werden der Reaktionsmischung 20 g einer 25%igen wäßrigen Tetramethylammoniumhydroxid-Lösung hinzugefügt. Der Siedepunkt am Kolonnenkopf erniedrigt sich, und es kann ein Gemisch aus Methanol und Methylacetat abdestilliert werden. Der Umsatz der TMP-Acetate ergibt sich aus der gaschromatographischen Analyse: TMP 43%, TMP-monoacetat 51%, TMP-diacetat 6%, TMP-triacetat 1%.

### Beispiel 1a)

Herstellung eines Gemisches der Ameisensäureester des 1,1,1-Trimethylolpropans (TMP).

134 g TMP werden mit 46 g Ameisensäure 5 Stunden zum Rückfluß erhitzt. Anschließend wird gebildetes Wasser abdestilliert. Zuletzt wird ein Gemisch aus TMP und den Mono-, Di- und Triformiaten von TMP abdestilliert. Die GC-Analyse ergibt ein Verhältnis von 49/40/10/1.

### Beispiel 1b)

81 g (0,5 Mol) des erhaltenen Gemisches aus a) werden in 64 g Methanol gelöst. Nach Zugabe von 4 g Triethylamin wird bei 40°C Sumpftemperatur gerührt. Nach 15 Minuten ergibt die GC-Analyse des Reaktionsgemisches eine Zusammensetzung von 80/18/2/-. Der Ansatz wird nun erhitzt, und am Kolonnenkopf wird Methylformiat abdestilliert, bis die Umsetzung nach einer Stunde quantitativ verlaufen ist.

### Vergleichsbeispiel 2

Es wird wie in Beispiel 1b) verfahren. Anstelle von Triethylamin werden 4 g 1,8-Diazabicyclo [5.4.0]undec-7-en (DBU) als Katalysator verwendet. Nach 5 Minuten sind 95% TMP und 5% TMP-Monoformiat vorhanden. Durch Abdestillieren von Methylformiat erreicht man quantitativen Umsatz.

### Beispiel 3

Es wird wie in Beispiel 1b) vorgegangen. Einsatzstoffe für die Umesterung sind 81 g TMP-Formiat-Gemisch (Beispiel 1a)), 80 g Methanol, 28,5 g N,N'-Dimethylpiperazin. Nach 2 Stunden können im Reaktionsgemisch keine TMP-Formiate mehr nachgewiesen werden.

### Beispiel 4

Es wird wie in Beispiel 1b) vorgegangen. Einsatzstoffe sind 81 g TMP-Formiat-Gemisch (Beispiel 1a)), 80 g Methanol, 28,5 g N-Ethylpiperidin. Nach 2 Stunden können im Reaktionsgemisch keine TMP-Formiate mehr nachgewiesen werden.

### Beispiel 5

Es wird wie in Beispiel 1b) vorgegangen. Einsatzstoffe sind 246 g TMP-Formiat-Gemisch (Beispiel 1a)), 370 g Ethanol, 76 g Triethylamin. Die Temperatur am Kolonnenkopf beträgt 54 - 55 °C (Sdp.: Ethylformiat). Es wird kontinuierlich abdestilliert. Nach 1 Stunde steigt die Temperatur auf 78°C an. Das Destillat enthält 88,7% Ethylformiat, 10,2% Ethanol, 1,1% Triethylamin. Der Umsatz bezüglich der TMP-Formiate ist 100%.

### Beispiel 6

Es wird wie in Beispiel 5 vorgegangen. Einsatzstoffe sind 246 g TMP-Formiat-Gemisch (Beispiel 1a)), 480 g Isopropanol, 76 g Triethylamin. Die Temperatur am Kolonnenkopf beträgt anfangs 72°C. Es wird kontinuierlich abdestilliert. Innerhalb von 4 Stunden steigt die Temperatur auf 82°C an. Das Destillat enthält 40% Isopropylformiat, 35% Isopropanol, 25% Triethylamin. Der Umsatz bezüglich der TMP-Formiate ist 100%

### Beispiel 7

Es wird wie in Beispiel 5 vorgegangen. Einsatzstoffe sind 66,5 g TMP-Formiat-Gemisch (Beispiel 1a)), 148 g tert.-Butanol, 19 g Triethylamin. Die Temperatur am Kolonnenkopf beträgt anfangs 77°C. Es wird kontinuierlich abdestilliert. Innerhalb von 1 Stunde steigt die Temperatur auf 82°C an. Das Destillat enthält 15% tert.-Butylformiat, 70% tert.-Butanol, 15% Triethylamin. Der Umsatz bezüglich der TMP-Formiate ist 100%.

### Beispiel 8

42 g eines Gemisches aus Cyclohexanol und Cyclohexylformiat (65/35) werden in 64 g Methanol gelöst. Nach Zugabe von 20 g Triethylamin wird zum Sieden erhitzt und über eine Kolonne kontinuierlich Methylformiat abdestilliert. Nach 4 Stunden beträgt das Verhältnis von Cyclohexanol zu Cyclohexylformiat 97/2,5.

### Beispiel 9

Es wird wie in Beispiel 8 vorgegangen, nur mit folgenden Einsatzstoffen: 15 g eines Gemischs aus tert.-Butanol und tert.-Butylformiat (97,8/1,2), 32g Methanol, 10 g Triethylamin. Nach 4 Stunden beträgt das Verhältnis von tert.-Butanol zu tert.-Butylformiat 99,1/0,9.

### Beispiel 10

Es wird wie in Beispiel 8 vorgegangen, nur mit folgenden Einsatzstoffen: 82,4 g eines Gemischs aus Hydroxypivalinsäureneopentylglykolester (HPN) und Hydroxypivalinsäureneopentylglykolestermono- und diformiat (94,5/3,2/2,3), 65 g Methanol, 4 g Triethylamin. Es wird 2 Stunden bei 40°C gerührt. Gaschromatographisch wird die Bildung von Methylformiat festgestellt. Die HPN-Komponenten liegen in einem Verhältnis von 98,8/1,2/- vor. Durch Abdestillieren von Methylformiat und Methanol wird vollständiger Umsatz bzgl. der HPN-Formiate erreicht. Es kann keine sonstige Nebenkomponente nachgewiesen werden.

### Vergleichsbeispiel 2

Es wird wie in Beispiel 1b) vorgegangen. Einsatzstoffe sind 243 g TMP-Formiat-Gemisch (Beispiel 1a)), 240 g Methanol, 135 g Wasser und 6 g NaOH. Der pH-Wert der Lösung sinkt im Laufe der Reaktion von 9,8 auf 5,3. Bei 65°C Sumpftemperatur wird bei einer Temperatur von 35°C am Kolonnenkopf Methylformiat abdestilliert. Nach 1 Stunde steigt die Kopftemperatur auf 65°C an. Die Zusammensetzung der Reaktionslösung ergibt ein Verhältnis von TMP und Mono-, Di- und Triformiaten von 83/16/1/-.

### Beispiel 11

Es wird wie in Vergleichsbeispiel 2 vorgegangen. Einsatzstoffe sind 243 g TMP-Formiat-Gemisch (Beispiel 1a)), 240 g Methanol, 135 g Wasser und 15 g Triethylamin. Der pH-Wert der Lösung sinkt im Laufe der Reaktion (3 Stunden) von 10,2 auf 5,3. Bei 65°C Sumpftemperatur wird bei einer Temperatur von 35°C am Kolonnenkopf Methylformiat abdestilliert. Nach 3 Stunden steigt die Kopftemperatur auf 65°C an. Die Zusammensetzung der Reaktionslösung ergibt ein Verhältnis von TMP und Mono-, Di- und Triformiaten von 96,5/3,5/-/-.

### Beispiel 12

Als Edukt eingesetztes PolyTHF-Monoformiat (hergestellt durch H₃PW₁₂O₄₀-katalysierte THF-Polymerisation in Gegenwart von Ameisensäure wie in EP-A-503 394 beschrieben wies folgende Kennzahlen auf:
Mₙ = 1030 (nach OH-Zahl und VZ);
VZ = 55 mg KOH/g; OH-Zahl = 54 mg KOH/g.

500 g PolyTHF-Monoformiat 1030 (0,5 mol) wurden in einer Destillationsapparatur in 480 g Methanol (15 mol) gelöst. Nach Zugabe von 5 g Triethylamin (0,05 mol; unter Argon zuvor destilliert) wurde das Reaktionsgemisch auf 67°C (Sumpftemperatur) erhitzt. Gebildetes Methylformiat (Kp. = 32°C) wurde über eine Vigreux-Kolonne abdestilliert, bis die Kopftemperatur 65°C betrug. Zur Entfernung von überschüssigem Methanol, Katalysator und niedermolekularen Oligomeren wurde der Rückstand im Vakuum bei 40°C eingeengt und anschließend bei 230°C/0,5 mbar kurz wegdestilliert.

Als Destillationsrückstand verbleibendes PolyTHF (440 g) wies folgende Kennzahlen auf:
Mₙ = 1170 (OH-Zahl);
OH-Zahl = 96 mg KOH/g; VZ = 0,3 mg KOH/g;
N-Restgehalt = 5 ppm (Bestimmungsmethode: Chemische Lumineszenz;
Nachweisgrenze ca. 1 ppm; Lit.: "Quantitative Organische Elementaranalyse", Friedrich Ehrenberger, VCH Weinheim, 1991, S. 382 ff; verwendetes Gerät: Fa. Dohrmann, DN 1000)
Nach Auswertung der VZ beträgt der Umsatz 99,5%;
Abkürzungen: OH-Zahl = Hydroxylzahl; VZ = Verseifungszahl;
Mₙ = mittleres Molekulargewicht (Zahlenmittel)

## Patentansprüche

1. Verfahren zur Herstellung von hydroxyhaltigen Verbindungen und Ameisensäureestern durch Umesterung, dadurch gekennzeichnet, daß man Ameisensäureester einer organischen hydroxyhaltigen Verbindung (1) und eine organische hydroxyhaltige Verbindung (2) in Gegenwart eines tertiären Amins ausgewählt aus tertiären Aminen a) der allgemeinen Strukturformel R^{a}R^{b}R^{c}N, wobei die Reste R^{a}, R^{b}, R^{c} gleich oder unterschiedlich sein können und jeweils unabhängig voneinander einen verzweigten oder nicht verzweigten Alkylrest mit vorzugsweise 1 bis 12, bevorzugter 1 bis 6, insbesondere 1 bis 3 Kohlenstoffatomen, Cycloalkylrest mit vorzugsweise 3 bis 12, insbesondere 6 bis 12 Kohlenstoffatomen oder aromatischen Kohlenwasserstoffrest mit vorzugsweise 6 bis 12 Kohlenstoffatomen bedeuten, welche gewünschtenfalls substituiert sein können, oder
b) heterocyclischen tertiären Stickstoffverbindungen ausgewählt aus Heterocycloalkan mit vorzugsweise 3 bis 12 Kohlenstoffatomen und 1 bis 3 Stickstoffatomen als Umesterungskatalysator unter Bildung der organischen hydroxyhaltigen Verbindung (1) und Ameisensäureestern der hydroxyhaltigen Verbindung (2) umestert, wobei Verbindungen (1) und (2) voneinander verschieden sind.

2. Verfahren nach Anspruch 1, dadurch gekennzeichnet, daß man die für die Umesterung zu verwendende hydroxyhaltige Verbindung (2) in bezug auf die Hydroxygruppen in mindestens stöchiometrischer Menge zum Ameisensäureester der hydroxyhaltigen Verbindung (1) in bezug auf die Ameisensäureestergruppen zugibt.

3. Verfahren nach Anspruch 1 oder 2, dadurch gekennzeichnet, daß man als Ameisensäureester einer hydroxyhaltigen Verbindung (1),
Ameisensäureester eines Polytetrahydrofurans, insbesondere den endständigen Monoameisensäureester, oder
Ameisensäureester des Trimethylolpropans, oder
Ameisensäureester der Hydroxypivalinsäureneopentylglykolester
oder Ameisensäureester eines Polyvinylalkohols
verwendet, wobei vorzugsweise sämtliche Ameisensäuregruppen entfernt werden.

4. Verfahren nach einem der Ansprüche 1 bis 3, dadurch gekennzeichnet, daß man bei der Umesterung als heterocyclische Stickstoffverbindung 1,4-Diazabicyclooctan, N,N'-Dialkylpiperazin, N-Alkylpiperidin oder N-Alkylaziridin verwendet.

5. Verfahren nach einem der Ansprüche 1 bis 4, dadurch gekennzeichnet, daß man das tertiäre Amin in einer Menge von 0,01 bis 10 Mol%, bevorzugt in einer Menge von 0,1 bis 5 Mol%, bezogen auf die Ameisensäureestergruppen als 100 Mol%, einsetzt.

6. Verfahren nach einem der Ansprüche 1 bis 5, dadurch gekennzeichnet, daß man für die Umesterung als hydroxyhaltige Verbindung solche mit 1 bis 6 OH-Gruppen, vorzugsweise ein-, zwei- oder dreiwertige, insbesondere einwertige Alkohole verwendet.

7. Verfahren nach einem der Ansprüche 1 bis 6, dadurch gekennzeichnet, daß man bei der Umesterung als organische hydroxyhaltige Verbindung (2) einen Alkohol mit der Formel R³OH, wobei R³ ein gesättigter oder ungesättigter, verzweigter oder nicht verzweigter, linearer Kohlenwasserstoffrest mit vorzugsweise 1 bis 20 Kohlenstoffatomen, insbesondere 1 bis 8 Kohlenstoffatomen, besonders bevorzugt 1 bis 3 Kohlenstoffatomen, ein aromatischer Kohlenwasserstoffrest mit vorzugsweise 6 bis 12 Kohlenstoffatomen oder ein Cycloalkylrest mit vorzugsweise 3 bis 12, insbesondere 6 bis 12 Kohlenstoffatomen ist, verwendet.

8. Verfahren nach einem der Ansprüche 1 bis 7, dadurch gekennzeichnet, daß man bei der Umesterung eine hydroxyhaltige Verbindung (2) verwendet, die leichter flüchtig ist als die bei der Umesterung gebildete hydroxyhaltige Verbindung (1).

9. Verfahren nach einem der Ansprüche 1 bis 8, dadurch gekennzeichnet, daß man bei der Umesterung eine hydroxyhaltige Verbindung (2) verwendet, deren Siedepunkt höher ist als der Siedepunkt des gebildeten Ameisensäureesters.

## Claims

1. A process for preparing hydroxyl-containing compounds and formic esters by transesterification, which comprises transesterification of formic esters of an organic hydroxyl-containing compound (1) and an organic hydroxyl-containing compound (2) in the presence of a tertiary amine selected from tertiary amines
a) of the general structural formula R^{a}R^{b}R^{c}N, where the radicals R^{a}, R^{b}, R^{c} can be identical or different and are each, independently of one another, a branched or unbranched alkyl radical having, preferably, 1 to 12, more preferably 1 to 6, in particular 1 to 3, carbon atoms, cycloalkyl radicals having, preferably 3 to 12, in particular 6 to 12, carbon atoms or aromatic hydrocarbon radical having, preferably, 6 to 12 carbon atoms, which may if desired be substituted, or
b) heterocyclic tertiary nitrogen compounds selected from heterocycloalkane having, preferably, 3 to 12 carbon atoms and 1 to 3 nitrogen atoms as transesterification catalyst to form the organic hydroxyl-containing compound (1) and formic esters of the hydroxyl-containing compound (2), where compounds (1) and (2) are different from one another.

2. A process as claimed in claim 1, wherein the hydroxyl-containing compound (2) to be used for the transesterification is added in terms of the hydroxyl groups in at least the stoichiometric amount relative to the formic ester of the hydroxyl-containing compound (1) in terms of the formic ester groups.

3. A process as claimed in claim 1 or 2, wherein the formic esters of a hydroxyl-containing compound (1) used are
formic esters of a polytetrahydrofuran, in particular the terminal monoformic ester, or
formic esters of trimethylolpropane, or
formic esters of neopentyl glycol hydroxypivalates
or formic esters of a polyvinyl alcohol,
and preferably all the formic acid groups are removed.

4. A process as claimed in any of claims 1 to 3, wherein the heterocyclic nitrogen compound used in the transesterification is 1,4-diazabicyclooctane, N,N'-dialkylpiperazine, N-alkylpiperidine or N-alkylaziridine.

5. A process as claimed in any of claims 1 to 4, wherein the tertiary amine is employed in an amount of from 0.01 to 10 mol%, preferably in an amount of from 0.1 to 5 mol%, based on the formic ester groups as 100 mol%.

6. A process as claimed in any of claims 1 to 5, wherein the hydroxyl-containing compound used for the transesterification is one with 1 to 6 OH groups, preferably monohydric, dihydric or trihydric, in particular monohydric, alcohols.

7. A process as claimed in any of claims 1 to 6, wherein the organic hydroxyl-containing compound (2) used for the transesterification is an alcohol having the formula R³OH, where R³ is a saturated or unsaturated, branched or unbranched, linear hydrocarbon radical having, preferably, 1 to 20 carbon atoms, in particular 1 to 8 carbon atoms, particularly preferably 1 to 3 carbon atoms, an aromatic hydrocarbon radical having, preferably, 6 to 12 carbon atoms or a cycloalkyl radical having, preferably, 3 to 12, in particular 6 to 12, carbon atoms.

8. A process as claimed in any of claims 1 to 7, wherein the hydroxyl-containing compound (2) used in the transesterification is more volatile than the hydroxyl-containing compound (1) formed in the transesterification.

9. A process as claimed in any of claims 1 to 8, wherein the hydroxyl-containing compound (2) used in the transesterification has a boiling point which is higher than the boiling point of the formic ester formed.

## Revendications

1. Procédé de fabrication de composés contenant des groupes hydroxyle et d'esters de l'acide formique, par transestérification, caractérisé en ce que l'on transestérifie des esters de l'acide formique d'un composé contenant des groupes hydroxyle (1) et un composé organique contenant des groupes hydroxyle (2) en présence d'une amine tertiaire choisie parmi
a) les amines tertiaires de formule de formule générale R^{a}R^{b}R^{c}N, dans laquelle les résidus R^{a}, R^{b}, R^{c} pouvant être identiques ou différents, représentent, indépendamment les uns des autres, un résidu alkyle ramifié ou non ramifié avec, de préférence, 1 à 12, plus particulièrement 1 à 6, tout particulièrement 1 à 3 atomes de carbone, un résidu cycloalkyle avec, de préférence, 3 à 12, en particulier 6 à 12 atomes de carbone, ou un résidu hydrocarbure aromatique avec, de préférence, 6 à 12 atomes de carbone qui peuvent éventuellement être substitués, ou
b) les composés hétérocycliques azotés à azote tertiaire choisis parmi les hétérocyclo-alcane avec, de préférence, 3 à 12 atomes de carbone et 1 à 3 atomes d'azote en tant que catalyseur de transestérification,
formant ainsi le composé organique contenant des groupes hydroxyle (1) et des esters de l'acide formique du composé contenant des groupes hydroxyle (2), les composés (1) et (2) étant alors différents l'un de l'autre.

2. Procédé selon la revendication 1 caractérisé en ce que l'on ajoute le composé contenant des groupes hydroxyle (2) à l'ester de l'acide formique du composé contenant des groupes hydroxyle (1), à mettre en oeuvre pour la transestérification une quantité au moins stoechiométrique des groupes contenant des groupes hydroxyle par rapport aux groupes esters de l'acide formique.

3. Procédé selon la revendication 1 ou 2, caractérisé en ce que l'on met en oeuvre en tant qu'ester de l'acide formique d'un composé contenant des groupes hydroxyle (1),
les esters de l'acide formique d'un polytétrahydrofuranne, en particulier les monoesters de l'acide formique d'extrémité de chaîne,
les esters de l'acide formique du triméthylolpropane, ou
les esters de l'acide formique des esters néopentylglycolique de l'acide hydroxypivalinique, ou
les esters de l'acide formique d'un poly(alcool vinylique),
tous les groupes de l'acide formique sont alors, de préférence, éliminés.

4. Procédé selon l'une des revendications 1 à 3, caractérisé en ce que, lors de la transestérification, on met en oeuvre, en tant que composé hétérocyclique azoté, le 1,4-bicyclooctanediazoïque, la N,N'-dialkylpipérazine, la N-alkylpipéridine ou la N-alkylaziridine.

5. Procédé selon l'une des revendications 1 à 4, caractérisé en ce que l'on met en oeuvre une amine tertiaire en une quantité allant de 0,01 à 10 % en mole, de préférence en une quantité allant de 0,1 à 5 % en mole, rapporté aux groupes ester de l'acide formique faisant 100% en mole.

6. Procédé selon l'une des revendications 1 à 5, caractérisé en ce que, pour la transestérification, on met en oeuvre en tant que composés contenant des groupes hydroxyle ceux possédant 1 à 6 groupes OH, de préférence les mono-, di- ou tétra-alcools, en particulier les monoalcools.

7. Procédé selon l'une quelconque des revendications 1 à 6, caractérisé en ce que l'on met en oeuvre, lors de la transestérification, en tant que composé organique contenant des groupes hydroxyle (2), un alcool de formule R³OH, R³ représentant alors un résidu hydrocarbure linéaire saturé ou insaturé, ramifié ou non ramifié ayant de préférence 1 à 20 atomes de carbone, en particulier 1 à 8 atomes de carbone, de manière plus particulièrement préférée 1 à 3 atomes de carbone, un résidu hydrocarbure aromatique avec, de préférence, 6 à 12 atomes de carbone ou un résidu cycloalkyle avec, de préférence, 3 à 12, en particulier 6 à 12 atomes de carbone.

8. Procédé selon l'une des revendications 1 à 7, caractérisé en ce que l'on met en oeuvre, lors de la transestérification, un composé contenant des groupes hydroxyle (2) qui est plus volatil que le composés contenant des groupes hydroxyle (1) formé lors de la transestérification.

9. Procédé selon l'une des revendications 1 à 8, caractérisé en ce que l'on met en oeuvre, lors de la transestérification, un composé contenant des groupes hydroxyle (2) dont le point d'ébullition est plus élevé que le point d'ébullition de l'ester de l'acide formique formé.
